# EUROPEAN PATENT SPECIFICATION

(11) **EP 0 684 074 B1**
(45) Date of publication and mention of the grant of the patent: **13.09.2000**
(21) Application number: 95105488.1
(22) Date of filing: 12.04.1995
(51) Int. Cl.: B01J 27/19, C07D 213/84, C07D 241/24

(54) **A solid catalyst for preparing nitriles and its preparation**
Fester Katalysator zur Herstellung von Nitrilen und Verfahren zu seiner Herstellung
Catalyseur solide pour le production de nitriles et son procédé de préparation

(30) Priority: 27.04.1994 KR 9408961
(43) Date of publication of application: 29.11.1995
(73) Proprietor: KOREA RESEARCH INSTITUTE OF CHEMICAL TECHNOLOGY, Taejon (KR)
(72) Inventor: Lee, Young K., Yusung-gu Taejon (KR); Shin, Chae-Ho, Junmin-dong, Yousung-ku, Daejeon 305-390 (KR); Chang, Tae-Sun, Yusung-gu, Taejon (KR); Cho, Deug-Hee, Yusung-gu, Taejon (KR); Lee, Dong-Ku, Yusung-gu, Taejon (KR)
(74) Representative: Weber, Dieter, Dr.

(56) References cited:
- EP-A- 0 113 156
- EP-A- 0 301 540
- US-A- 4 444 906

## Description

### FIELD OF THE INVENTION

This invention relates to a solid catalyst for preparing nitriles and its preparation which can improve the conversion and the selectivity when used as a catalyst during the process for preparing heteroaromatic nitriles.

In the preparation of heteroaromatic nitriles, such as cyanopyrazine or cyanopyridine, by ammoxidation of an alkyl-substituted heteroaromatic compound, vanadium oxide [Applied Catalysis, Vol. 83, p 103 (1992)], vanadium oxide/alumina or their mixture with the other various metal oxide are used as a catalyst.

It is known that transition metal oxides, such as antimony oxide, molybdenum oxide, iron oxide and tin oxide, have the activity for ammoxidation of an alkyl-substituted heteroaromatic compound.

Besides, multi-element oxide such as antimony/vanadium/magnanese oxide supported in alumina are often used as catalyst.

These methods are known to give a conversion of the reactant of 90% and a selectivity for cyanopyrazine of 75% [Applied Catalysis, Vol. 20, p219 (1986)].

On the other hand, reactivities of alkyl-substituted heteroaromatic compounds were analyzed on "Applied Catalysis, Vol. 85, p47" as followings; when Cu-Na / zeolite catalyst is used, the conversion of 3-methylpyridine is 82%, and the selectivity for pyridine is 4% and for 3-cyanopyridine is 96%. In 1986, Shimiz and Shinkichi et al. disclosed that when VPₓSb_{y}O_{z} catalyst is used, the conversion of 3-methylpyridine is 98.7% and the selectivity for 3-cyanopyridine is 80% (EP-A-253 360). But, the above literature does not disclose the selectivities for pyridine, tar, carbon monoxide, carbon dioxide, etc.

In 1979, A. Anderson disclosed that when V₆O₁₃ as catalyst is used, the conversion of 3-methylpyridine is 100%, the selectivity for 3-cyanopyridine is 80%, the selectivities for carbon monoxide and carbon dioxide are 15% and the selectivity for tar is 5% [Journal of Catalyst, Vol. 58, p283].

In 1989, Anderson disclosed that when vanadium-titanium-oxygen catalyst is used, the conversion of 3-methylpyridine is 100%, the selectivity for 3-cyanopyridine is 80%, the selectivities for carbon monoxide and carbon dioxide are 5% and the selectivity for tar is 15% [Journal of Catalyst, Vol. 65, p9].

Finally, in 1989, A. Martin et al. disclosed that when vanadium-phosphorus-oxygen catalyst is used, the conversion of 3-methylpyridine is 98%, the selectivity for 3-cyanopyridine is 93% and the selectivities for carbon monoxide and carbon dioxide are 15% [Applide Catalyst, Vol. 49, p205].

The conversion of 3-methylpyridine and the selectivities of 3-cyanopyridine by using the above known catalysts may be compared as followings ; when the conversion of 3-methylpyridine is 80 to 100 %, the selectivity for 3-cyanopyridine is about 80 %. As the result it was found that side reactions occured under the above catalysts and simultaneous by-product of a great quantity is produced. Above, the cases of 2-methylpyrazine and 3-methylpyridine were illustrated but the cases of 2-methylpyridine and 4-methylpyridine were also analyzed to give similar result.

The EP-A-0 113 156 discloses catalysts containing Mo, P and O for the production of unsaturated carbocyclic acids. The catalysts are calcined within a gas containing 0,05 to 3 % of ammonia and/or steam. The EP-A-0 301 540 shows a process for preparing heteroaromatic nitriles in the presence of a catalyst which may contain Mo, P and O, but no NH₄ ions.

Ammoxidation of an alkyl-substituted heteroaromatic compound is carried out by the following process; cyanopyrazine or cyanopyridine as the desired compound is obtained through acid and amide as the intermediates [Int. Chem. Eng. 8, 888 (1968)], and then the selectivity of cyanoprazine or cyanopyridine is closely related to those of acid and amide. Commonly, the more the selectivities of cyanopyrazine and cyanopyridine, as the major reaction product of methylpyrazine or methylpyridine, are increased, the more those of acid and amide are decreased. On the other hand, the more the selectivities of cyanopyrazine and cyanopyridine are decreased, the more those of acid and amide are increased.

The inventors investigated about new catalyst with controllable production of by-products by increasing the conversion and the selectivity during the process for preparing a heteroaromatic nitriles. As the result, it is found that a catalyst comprising molybdenum and phosphorus with proper ratio as main component has high activity and selectivity, in particular that the thermal stability or hygroscopic property at atmosphere and reaction activity are greatly changed in accordance with content of phosphorus.

### SUMMARY OF THE INVENTION

The object of this invention is to provide a solid catalyst for preparing nitriles which can effectively control the production of by-product by increasing the conversion and the selectivity during the process for preparing a heteroaromatic nitrile by ammoxidation of an alkyl-substituted heteroaromatic compound in the vapor phase. This invention relates to a solid catalyst for preparing nitriles, having the following formula

Moₓ · P_{y} · O_{z} · X · Y

wherein,
Mo is molybdenum;
P is phosphorus;
O ix oxygen;
X is one or several ammonium ions;
Y is one or several mols water; and
x, y and z are respectively the number of atoms of Mo, P and O, wherein y/x is 0.01 to 5 and z is 0.01 to 10, and having been activated by heating at 400 to 600°C under nitrogen gas.

### DETAILED DESCRIPTION OF THE INVENTION

This invention relates to a novel solid catalyst which can improve the conversion and the selectivity in the process for preparing heteroaromatic cyanides by ammoxidation of an alkyl-substituted heteroaromatic compounds, and its preparation. According to this invention, the solid catalyst can be prepared by the following process.

The catalyst of the invention can especially be considered as a reaction product of a molybdenum containing ammonium salt with sixth an amount of phosphoric acid, that the atomic ratio of phosphorus to molybdenum in the reaction product is 0,01 to 5.

Firstly, a salt containing molybdenum, for example ammonium molybdate tetrahydrate ((NH4)₆Mo₇O₂₄ · 4H₂O), is added to water, and completely dissolved at the maximum temperature of 100 °C by slowly increasing the reaction temperature. Phosphoric acid is added to the solution in order that y/x, the atomic ratio of phosphorus to molybdenum, is 0,01 to 5. If y/x is less than 0.01, the desired reaction activity cannot be obtained, and if y/x is more than 5, the hygroscopic property of catalyst itself is too high because of addition of excessive phosphoric acid and the reactivity is decreased. Therefore, in this invention, in order to accelerate the reaction rate and easily remove residue, the temperature of the above reaction solution should be raised by 50 to 100 °C, and then water is removed during the reaction under stirring to concentrate the reactant, thereby the desired product is obtained as the bulk solid.

According to this invention, the bulk solid may be dried for 1 to 15 hours in an oven of 30 to 150 °C and pulverized to be effectively used as a catalyst. The pulverized powder is kept in an airtight dessicator to prevent from contacting with water in atmosphere. The more y/x of the pulverized powder is decreased, the more the color is white, and the more y/x is increased, the more the color is light green and the hygroscopic property is increased.

The solid catalyst of this invention prepared by the above method was confirmed by BET surface area measurement, pore volume measurement, infrared spectrometer, thermal gravimetric spectrometer, X-ray diffraction spectroscope, X-ray fluorescence spectroscope, elemental analyzer, analysis of molybdenum, phosphorus, etc. According to the result of thermal gravimetric analysis for the catalyst, the catalyst weight was decreased at 350°C under nitrogen by removing ammonium salt or ion(x), water(y) and other impurity.

The solid catalyst prepared by the above method is pretreated and activated at 400 to 600°C under nitrogen, in order to effectively use it as a reaction catalyst. According to the result of X-ray diffraction analysis, it is found that a phase having thermal stability exists as amorphous form.

To activate the catalyst, a quartz reactor with a sintered quartz is filled with catalyst, and then nitrogen gas is passed in at 4 l/hr of flow and pretreatment is carried out at 400 to 600 °C for 2 to 6 hours. As the result, the conversion and the selectivity of the activated catalyst were improved in comparison with those of the known catalyst. The solid catalyst of this invention has a high activity in the process for preparing a heteroaromatic nitrile.

This invention may be illustrated in more detail by the following examples, but it is not limited by the examples.

### Example 1.

18,54 g ammonium molybdate tetrahydrate [(NH₄)₆Mo₇O₂₄ · 4H₂O] of were dissolved in 100 *ml* of water, and the solution was slowly heated under stirring. After adding 3.63 g of 85 % phosphoric acid (y/x = 0.3), the solution was stirred at 80 °C and concentrated to remove water.

The obtained bulk solid was completly dried in an oven and pulverized, and then the final product was kept in a dessicator to prevent from contacting with water.

### Example 2.

A catalyst was prepared by using the method discribed in the above Example 1 except that 200 *ml* of water and 9.08 g of 85 % phosphoric add (y/x = 0.75) were used.

### Example 3.

A catalyst was prepared by using the method described in the above Example 1 except that 12.11 g of 85 % phosphoric acid (y/x = 1.0) was used.

### Example 4.

A catalyst was prepared by using the method described in the above Example 1 except that 18.16 g of 85 % phosphoric acid (y/x = 1.5) was used.

### Comparative Example 1.

A catalyst was prepared by using the method described in the above Example 1 except that 72.66 g of 85 % phosphoric acid (y/x = 6.0) was used.

### Comparative Example 2.

A catalyst was prepared by using the method described in the above Example 1 except that 0.06 g of 85 % phosphoric acid (y/x = 0,005) was used.

### Experimental Example.

In order to activate catalysts prepared by Examples 1 to 4 and Comparative Examples 1 to 2, a quartz reactor (16 mm diameter, 200 mm length) consisting of sintered quartz was filled with 500 mg of prepared catalyst and then pretreated at 500 °C. Nitrogen gas was passed into the reactor at a flow rate of 4 *l*/hr for 4 hours

After adding an alkyl-substituted heteroaromatic compound such as 2-methyl pyrazine, 2-methylpyridine, 3-methylpyridine , 2-methylpyridine, 3-methylpyridine or 4-methylpyridine, 10 moles of ammonia and 13.5 moles of oxygen were added per 1 mole of an alkyl-substituted heteroaromatic compound, and then ammoxidation was carried out for 24 hours at 400 °C,

The result was given in Table 1.

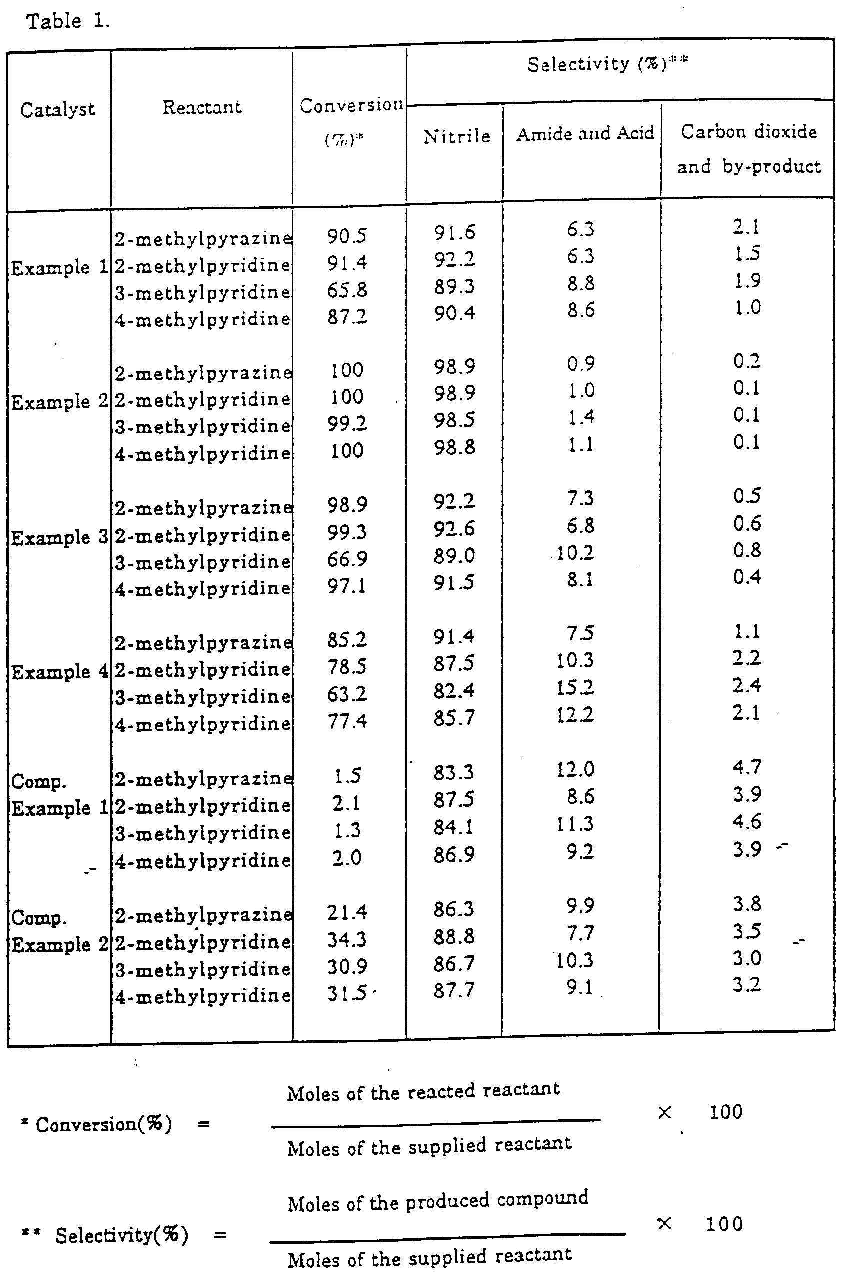

As the above results show the conversion and the selectivity are quite different for each catalyst. When a heteroaromatic nitrile was prepared by a catalyst of this invention, the selectivity of by-product such as carbon dioxide and tar, etc. was below 1 % as the sum of all, and the conversion was increased up to 100 %. The catalyst prepared by this invention has shown, a high activity for preparing a heteroaromatic nitrile.

## Claims

1. A solid catalyst for preparing nitriles having the following composition
Moₓ . P_{y} . O_{z} . X . Y
wherein
Mo is molybdenum;
P is phosphorus;
O is oxygen;
X is one or several ammonium ions;
Y is one or several mols water; and
x, y and z are respectively the number of atoms of Mo, P and O, wherein y/x is 0.01 to 5 and z is 0,01 to 10, and having been activated by heating at 400 to 600 °C under nitrogen gas.

2. A method for preparing a solid catalyst according to claim 1, comprising the steps of adding molybdenum salt into water, dissolving at from room temperature to 100 °C, adding phosphoric acid under stirring, obtaining solid precipitate during the reaction, drying and pulverizing the obtained bulk solid and activating it at 400 to 600 °C under nitrogen gas.

3. The method according to claim 2, wherein said molybdenum salt is ammonium molybdate tetrahydrate [(NH₄)₆Mo₇O₂₄ . 4H₂O].

4. The method according to claim 2, wherein said molybdenum salt and said phosphoric acid are added in the atomic ratio of phosphorus to molybdenum of from 0,01 to 5.

5. Use of a solid catalyst according to claim 1 for preparing a heteroaromatic nitrile by ammoxidation of an alkyl-substituted heteroaromatic compound.

6. The use of the solid catalyst to claim 5 for the ammoxidation of 2-methylpyrazine, 2-methylpyridine, 3-methylpyridine or 4-methylpyridine.

## Patentansprüche

1. Fester Katalysator zur Herstellung von Nitrilen mit der folgenden Zusammenetzung:
Moₓ · P_{y} · O_{Z} · X · Y
worin
Mo Molybdän ist,
P Phosphor ist,
O Sauerstoff ist,
X ein oder mehrere Ammoniumionen ist,
Y ein oder mehrere Mole Wasser ist und
x, y und z jeweils die Anzahl von Atomen von Mo, P und O sind, wobei y/x 0,01 bis 5 ist und z 0,01 bis 10 ist, und der durch Erhitzen auf 400 bis 600 °C unter Stickstoffgas aktiviert wurde.

2. Verfahren zur Herstellung eines festen Katalysators nach Anspruch 1 mit den Stufen, in denen man Molybdänsalz zu Wasser zusetzt, bei einer Temperatur von Raumtemperatur bis 100 °C auflöst, Phosphorsäure unter Rühren zugibt, einen festen Niederschlag während der Umsetzung erhält, den erhaltenen voluminösen Feststoff trocknet und pulverisiert und ihn bei 400 bis 600 °C unter Stickstoffgas aktiviert.

3. Verfahren nach Anspruch 2, bei dem das Molybdänsalz Ammoniummolybdattetrahydrat [(NH₄)₆Mo₇O₂₄ · 4H₂O] ist.

4. Verfahren nach Anspruch 2, bei dem man das Molybdänsalz und die Phosphorsäure im Atomverhältnis von Phosphor zu Molybdän von 0,01 bis 5 zusetzt.

5. Verwendung eines festen Katalysators nach Anspruch 1 zur Herstellung eines heteroaromatischen Nitrils durch Ammonooxidation einer alkylsubstituierten heteroaromatischen Verbindung.

6. Verwendung des festen Katalysators nach Anspruch 5 zur Ammonooxidation von 2-Methylpyrazin, 2-Methylpyridin, 3-Methylpyridin oder 4-Methylpyridin.

## Revendications

1. Catalyseur solide pour la préparation de nitriles ayant la composition suivante
Moₓ · P_{y} · O_{z} · X · Y
où
Mo est le molybdène;
P est le phosphore;
O est l'oxygène;
X est un ou plusieurs ions ammonium;
Y est une ou plusieurs moles d'eau; et
x, y et z sont respectivement le nombre d'atomes de Mo, P et O, où y/x vaut de 0,01 à 5 et z vaut de 0,01 à 10, et ayant été activé par chauffage à 400 à 600 °C sous azote gazeux.

2. Procédé de préparation d'un catalyseur solide selon la revendication 1, comprenant les étapes consistant à ajouter du sel de molybdène dans de l'eau, à dissoudre à partir de la température ambiante jusqu'à 100°C, à ajouter de l'acide phosphorique sous agitation, à obtenir un précipité solide durant la réaction, à sécher et à pulvériser le solide en vrac obtenu et à l'activer à 400 à 600°C sous azote gazeux.

3. Procédé selon la revendication 2, dans lequel ledit sel de molybdène est le molybdate d'ammonium tétrahydraté [(NH₄)₆Mo₇O₂₄.4H₂O].

4. Procédé selon la revendication 2, dans lequel ledit sel de molybdène et ledit acide phosphorique sont ajoutés dans un rapport atomique du phosphore au molybdène de 0,01 à 5.

5. Utilisation d'un catalyseur solide selon la revendication 1 pour préparer un nitrile hétéroaromatique par ammoxydation d'un composé hétéroaromatique alkylé.

6. Utilisation du catalyseur solide selon la revendication 5 pour l'ammoxydation de la 2-méthylpyrazine, de la 2-méthylpyridine, de la 3-méthylpyridine ou de la 4-méthylpyridine.
